# EUROPEAN PATENT APPLICATION

(11) **EP 1 053 677 A1**
(43) Date of publication of application: **22.11.2000**
(21) Application number: 99902843.4
(22) Date of filing: 05.02.1999
(51) Int. Cl.: A01K 67/027, C12N 5/06, C12N 15/12

(54) **NONHUMAN ANIMAL EMBRYONIC STEM CELLS AND NONHUMAN ANIMALS HAVING VARIANT SMOOTH MUSCLE MYOSIN HEAVY-CHAIN GENES**

(30) Priority: 05.02.1998 JP 3975298; 10.03.1998 JP 7658398
(71) Applicant: Vessel Research Laboratory Co., Ltd., Machida-shi, Tokyo 194-8533 (JP)
(72) Inventor: HARIGAI, Takashi, Vessel Res. Lab. Co., Ltd., Machida-shi, Tokyo 194-8533 (JP); HASEGAWA, Kazuhide, Vessel Res. Lab. Co., Ltd., Machida-shi, Tokyo 194-8533 (JP); SUGIYAMA, Tomoyasu, Vessel Res. Lab. Co., Ltd., Machida-shi, Tokyo 194-8533 (JP); KUROO, Makoto, Higashimurayama-shi, Tokyo 189-0012 (JP); NABESHIMA, Youichi, Yono-shi, Saitama 338-0013 (JP); NAGAI, Ryozo, Maebashi-shi, Gunma 371-0033 (JP)
(74) Representative: Lee, Nicholas John
(86) International application number: JP9900505
(87) International publication number: WO9939571

(57) **Abstract**

The cells and the non-human animal are disclosed, which can provide a new study material to be used in biomedical study concerning a role, a physiological function, and a mechanism for the expression of a smooth muscle myosin heavy chain gene as in the blood vessel, also provide a test system for the evaluation of a pharmaceutical preparation against arteriosclerosis and the like, and is useful for the identification of cells expressing a smooth muscle myosin heavy chain. The cells, and non-human animal and the progeny thereof contain a variant gene which is obtained by deleting a part or whole of a smooth muscle myosin heavy chain gene derived from a chromosome (SM1 or SM2), by inserting or substituting a foreign gene, whereby the variant gene can not substantially function as the smooth muscle myosin heavy chain gene.

## Description

### Technical Field

This invention relates to an animal of a novel line, a method for the production thereof, and novel embryonic stem cells to be used for the production thereof. More particularly, this invention relates to an animal having a mutated myosin heavy chain gene of a smooth muscle and befitting adoption as a new research material for the biomedical study of a physiological function and a gene expression mechanism of a smooth muscle myosin heavy chain in a blood vessel and as an experimental animal useful for the study of pathologic physiology of cardiovascular diseases and the development of therapeutic agents for the diseases, a method for the production of the animal, and novel embryonic stem cells to be used for this production.

### Background

The interesting characteristics of smooth muscle cells consists not only in the fact that a myofilament existing therein is quantitatively changed depending on a cytoplasm but also in the fact that molecular species of a structural protein forming the myofilament changes by a phenomenon which is called "isoform switching". A myosin heavy chain of smooth muscle cells in a blood vessel expresses two kinds of isoforms, SM1 and SM2, in the differentiated smooth muscle cells, which isoforms are generated by alternative splicing from one chromosome gene (SM1 gene). An undifferentiated smooth muscle in a fetal period expresses an isoform generated from another chromosome gene called "SMemb" together with SM1. An important point is that an isoform of a myosin heavy chain is considered to be useful as an index for differentiation of a smooth muscle.

A myosin heavy chain gene of a smooth muscle which is related to the isoform of myosin heavy chain has been reported, for example, with respect to a rabbit cDNA (Nagai et al., The Journal of Biological Chemistry, Vol. 264, No. 17, pp. 9734-9737, 1989) and a murine cDNA (Hasegawa et al., Biochemical and Biophysical Research Communications, Vo1. 232, pp. 313-316, 1997). No animals having a myosin heavy chain gene of a smooth muscle deleted have been heretofore known to the art.

As regards cultured cells of a smooth muscle, a cell line having a SV40 large T-antigen gene introduced therein has been established (Hasegawa et al., Journal of Molecular and Cellular Cardiology, Vol. 29, pp. 2177-2186, 1997). No cell lines having the function thereof lost by the mutation in the myosin heavy chain gene of the smooth muscle of chromosome thereof have never been known to the art.

It is naturally estimated that a cell or an animal which is deprived of the function of the smooth muscle myosin heavy chain gene ought to be greatly useful in studying a physiological function and a gene expression mechanism of a smooth muscle myosin heavy chain in a blood vessel and further in studying pathologic physiology of cardiovascular disease originating in the smooth muscle myosin heavy chain and developing a therapeutic agent therefor. In spite of this prospect, no cells and animals in question have been developed to date but the desirability of this development has been finding veritably enthusiastic recognition.

This invention, therefore, has an object to provide a variant animal which contains a variant gene substantially deprived of a function of a smooth muscle myosin heavy chain gene derived from a chromosome by the manipulation of the smooth muscle myosin heavy chain gene using a gene manipulating technique, and embryonic stem cells which can be applied to the production and differential experiment of the variant animal as described above.

This invention has another object to provide a method for producing an animal having a smooth muscle myosin heavy chain gene mutated by the use of the embryonic stem cells.

This invention has still another object to provide cells of a genetic background having a mutation in a smooth muscle myosin heavy chain gene by culturing the embryonic stem cells or the cells from the variant animal.

### Disclosure of the Invention

The present inventors have made a diligent study in order to solve the problems mentioned above, to succeed in artificially producing embryonic stem cells and an animal which contains a smooth muscle myosin heavy chain gene incapable of substantially functioning and expresses a reporter gene in place of the native smooth muscle myosin heavy chain gene by inducing a homologous recombination between a smooth muscle myosin heavy chain gene derived from a murine chromosome and a vector DNA designed and prepared for the homologous recombination and inserting a different sequence in the DNA sequence of the gene mentioned above. This invention has been perfected as a result.

They have further succeeded in differentiating cells having the mutation induced during the production of the animal or acquiring novel cells by culturing the cells from the produced animal. This invention has been also perfected as a result.

To be specific, this invention involves the features given in following items (1) to (15).
(1) Embryonic stem cells of a non-human animal or the cells derived therefrom which contain a variant smooth muscle myosin heavy chain gene incapable of substantially functioning as a smooth muscle myosin heavy chain gene derived from a chromosome.
(2) Embryonic stem cells or the cells derived therefrom according to the item (1) mentioned above, wherein the variant gene is obtained by deleting at least a part of the base sequence for the smooth muscle myosin heavy chain gene derived from the chromosome, by inserting a foreign gene into the smooth muscle myosin heavy chain gene derived from the chromosome, by substituting at least a part of the smooth muscle myosin heavy chain gene derived from the chromosome with a foreign gene, or by the combinations thereof, whereby the function of the smooth muscle myosin heavy chain gene derived from the chromosome is substantially lost.
(3) Embryonic stem cells or the cells derived therefrom according to the item (2) mentioned above, wherein the variant gene is obtained by inserting a reporter gene in the smooth muscle myosin heavy chain gene derived from the chromosome.
(4) Embryonic stem cells or the cells derived therefrom according to the item (3) mentioned above, wherein the reporter gene is a β-galactosidase gene.
(5) Embryonic stem cells or the cells derived therefrom according to any one of the items (1) to (4) mentioned above, wherein the non-human animal is a rodent.
(6) Embryonic stem cells or the cells derived therefrom according to the item (5) mentioned above, wherein the rodent is a mouse.
(7) A non-human animal or the progeny thereof which contains a variant smooth muscle myosin heavy chain gene incapable of substantially functioning as a smooth muscle myosin heavy chain gene derived from a chromosome.
(8) A non-human animal or the progeny thereof according to the item (7) mentioned above, wherein the variant gene is obtained by deleting at least a part of the base sequence for the smooth muscle myosin heavy chain gene derived from the chromosome, by inserting a foreign gene into the smooth muscle myosin heavy chain gene derived from the chromosome, by substituting at least a part of the smooth muscle myosin heavy chain gene derived from the chromosome with a foreign gene, or by the combinations thereof, whereby the function of the smooth muscle myosin heavy chain gene derived from the chromosome is substantially lost.
(9) A non-human animal or the progeny thereof according to the item (8) mentioned above, wherein the variant gene is obtained by inserting a reporter gene in the smooth muscle myosin heavy chain gene derived from the chromosome.
(10) A non-human animal or the progeny thereof according to the item (9) mentioned above, wherein the reporter gene is a β-galactosidase gene.
(11) A non-human animal or the progeny thereof according to any one of the items (7) to (10) mentioned above, wherein the non-human animal is a rodent.
(12) A non-human animal or the progeny thereof according to the item (11), wherein the rodent is a mouse.
(13) Cells which are obtained by separating and culturing from the non-human animal or the progeny thereof set forth in any one of the items (9) to (12).
(14) A cell line which is obtained by introducing a T antigen gene into the cells set forth in the item (13) mentioned above.
(15) A cell line according to the item (14) mentioned above, which is a myeloma cell line deposited as "SM1BMC #1" under the accession No. FERM BP-6625.

### Brief Description of the Drawings

Fig. 1 is a schematic diagram of a primer used in the strategy and PCR of homologous recombination for mutating a murine smooth muscle myosin heavy chain gene and a probe used for Southern blotting.
Fig. 2 is a diagram illustrating results of analysis by Southern blotting of ES cells chromosome DNA resulted from homologous recombination.
Fig. 3 is a photograph showing results by PCR indicating that the chromosome of the SM1BMC #1 contains a variant gene resulting from homologous recombination.
Fig. 4 shows the number of β-galactosidase positive cells in a laminin-coated culture plate.

### Best Modes for Carrying Out the Invention

The non-human embryonic stem cells or the cells derived therefrom of the present invention is characterized by containing a variant smooth muscle myosin heavy chain gene (referred to occasionally simply as "variant gene") which is incapable of substantially functioning as a smooth muscle myosin heavy chain gene derived from a chromosome (referred to occasionally simply as "wild-type gene").

Now, this invention will be described in detail below.

The expression "the variant gene is substantially incapable of functioning as a smooth muscle myosin heavy chain gene derived from a chromosome" as used in this invention means that a generic product (a translated product) of a variant gene can not be obtained as a protein having a structure identical to a wild-type smooth muscle myosin heavy chain derived from a chromosome. To be specific, "a variant gene which is incapable of substantially functioning as a smooth muscle myosin heavy chain gene derived from a chromosome" may be obtained by gene manipulating means, for example, by deleting at least a part of the base sequence of the gene or inserting a foreign gene or substituting a foreign gene therefor. As typical examples of the variant gene incapable of substantially functioning, a variant gene having a promoter broken by the deletion or substitution of the base sequence of a promoter region thereby losing the transcription activity, a variant gene which can not provide a stable transcribed product by the deletion or substitution of the base sequence of a non-translation region, a variant gene which can not provide a gene product by the deletion or substitution of the base sequence of a translation region coding for the gene product or by the insertion of another gene, and a variant gene which can not serve as a smooth muscle myosin heavy chain although the gene product can be obtained may be cited.

Then, the expression "embryonic stem cells of a non-human animal containing a variant gene incapable of substantially functioning as a smooth muscle myosin heavy chain gene derived from a chromosome" as used in the present invention refers to embryonic stem cells of a non-human animal which function as a smooth muscle myosin heavy chain gene is substantially lost by artificially mutating a smooth muscle myosin heavy chain genome gene as described above. As typical examples of such embryonic stem cells of the non-human animal, embryonic stem cells (ES cells) of a non-human animal the variant cells of which is substantially disabled to function as a smooth muscle myosin heavy chain gene by the insertion of a different gene into the smooth muscle myosin heavy chain gene may be cited.

Further, the expression "the cells derived therefrom" as used in this invention means cells which is as a source the embryonic stem cells according to this invention such as,for example,a cell line obtained by culturing the embryonic stem cells according to this invention and cells to be obtained by inducing the differentiation of the embryonic stem cells according to this invention. In this case, the expression "cells to be obtained by inducing the differentiation of the embryonic stem cells" as used herein refers to cells which is differentiated into cells of various types such as of longus capitis muscle, visceral muscle, and cardiac muscle, for example, by continuing the monolayer culture to attain a high density or continuing the suspension culture until a cell cluster is formed, for example, under proper conditions (Experimental Medicine, Bio Manual Series as a separate-volume supplement, Vol. 8, page 147, written by Shinichi Aizawa and published by Yodosha K.K.). The cells containing such a variant myosin heavy chain gene as obtained in the manner described above is useful in testing an in vitro test on the action and function of a smooth muscle myosin heavy chain gene or a protein.

The non-human animal of this invention involves both an animal having a smooth muscle myosin heavy chain gene heterologously mutated and an animal having this gene homologously mutated. The non-human animal of this invention further involves a chimera animal having a smooth muscle myosin heavy chain gene heterologously or homologously deleted in a part of the structural cells thereof.

The non-human animal to be used in this invention does not need to be particularly limited but is only required to be capable of producing a chimera animal. It is applicable to all the animals other than human such as, for example, mammals, birds, amphibians, and fishes. Actually, in consideration of the industrial applicability, however, such a mammal as rabbit, pig, sheep, goat, cattle, rat, and mouse are preferably used. Further in respect of ontogenesis, a short life cycle, and relatively easy propagation, such rodents as mouse and rat, particularly mouse, are used particularly preferably in producing an animal model.

As one way of obtaining the cells of this invention, a method by which cells containing a smooth muscle myosin heavy chain gene having its function substantially lost by means of mutation procedure is produced may be available. When the produced cells are embryonic stem cells, for example, the animal according to this invention can be obtained by producing a chimera animal from these cells. Further, by using this chimera animal, it is made possible to produce an animal which has the smooth muscle myosin heavy chain gene mutated heterologously or homologously in all the structural cells thereof. It is also possible to produce cells by isolating and culturing cells from the animal containing such a produced variant gene.

In this invention, as typical examples of the method for obtaining a smooth muscle myosin heavy chain gene incapable of substantially functioning, a method which comprises deleting at least a part of a base sequence of a smooth muscle myosin heavy chain gene derived from a chromosome, a method which comprises partly substituting the base to generate a terminal codon, a method which comprises inserting a foreign gene into a smooth muscle myosin heavy chain gene derived from a chromosome, and a method which comprises substituting at least a part of a smooth muscle myosin heavy chain gene derived from a chromosome with a foreign gene, i.e. methods which have already known in the art, may be cited. The site for deletion, insertion, or substitution in this case is not particularly discriminated but is only required to permit the substantial deficiency of the function from the produced smooth muscle myosin heavy chain gene. The targeting site may be any of a promoter region, a non-translation region, or a translation region of a smooth muscle myosin heavy chain gene. The targeting may be either at one site or at a plurality of sites. When the targeting is positioned at a plurality of sites, the gene manipulating means may be either identical or not identical at each of the sites. For the purpose of losing the function of a gene efficiently and completely, however, a translation region is preferably targeted.

In the methods which involve the insertion or substitution of a foreign gene in a wild-type gene among other methods cited above, as typical examples of the foreign gene, a reporter gene and a drug-resistance gene such as a neomycin-resistant gene (neo), herpes virus thymidine phosphorylated enzyme gene (HSV-tk), and diphtheria toxin A fragment gene (DT-A) may be cited. Among other foreign genes, a reporter gene is advantageously inserted in a wild-type gene. The term "a reporter gene" as used herein refers to a gene which is linked to a downstream of a promoter of a given gene for the purpose of detecting the expression of the promoter of the gene or determining the intensity thereof. The gene, for example, of an enzyme which is not detected internally, and is stable and easy to be determined may be used. When a variant gene is made by inserting a reporter gene in a wild-type gene, for example, since the cells which express the gene can be visually detected by using as an index the expression of the reporter gene (for example, the development of color as a substrate in the case of using lacZ as the reporter gene),the presence or absence of the expression can be easily detected, which is advantageous. Accordingly, the use of the reporter gene in the case of a heterozygous animal carrying a variant gene can allow the detection of expressed cells using the expression of the reporter gene as an index and, at the same time, is expected to permit the study for the control of the expression of a functional (non-variant type) smooth muscle myosin heavy chain gene maintained on a gene locus of another chromosome. In the case of a homozygous animal, expressed cells can be detected using the expression of the reporter gene as an index and, at the same time, it is expected that the physiological function and the change in an expression-controlling gene can be studied when the smooth muscle myosin heavy chain genes are deleted at both the gene loci. Additionally, the use of a reporter gene is expected to become a testing system convenient for studying biological and medical utilities or effects of a compound because it permits simple detection of the change in the expression of a smooth muscle myosin gene relative to the compound.

The reporter gene which is usable in this invention is not particularly limited but is only required to be commonly used. Typically, β-galactosidase gene (lacZ) from E. coli, chloramphenicol acetyl transferase gene (CAT) from a bacteria transposon, luciferase gene (Luc) from Lampyridae, and green fluorescent protein gene (GFP) from jellyfish may be cited. Among other reporter genes, β-galactosidase gene (lacZ) from E. coli is preferably used from the point of view that histological analysis can be easily carried out by the use of a substrate which colors in visible light.

In this invention, the means for producing cells containing a smooth muscle myosin heavy chain gene incapable of substantially functioning is not particularly discriminated but is preferred to have resulted from homologous recombination.

Although the homologous recombination technique has been already well-known in the art and is not particularly discriminated, it may be carried out as follows. Firstly, a smooth muscle myosin heavy chain gene derived from a chromosome of an animal species which is expected to be mutated into a variant smooth muscle myosin heavy chain gene may be isolated by a conventional method. In this case, as means for isolating a smooth muscle myosin heavy chain gene of a chromosome, a method which obtains a clone of a smooth muscle myosin heavy chain gene of a chromosome containing a non-translation region by screening genome DNA libraries as by plaque hybridization using as a probe a part of a smooth muscle myosin heavy chain gene having the base sequence already reported or a part of the cDNA may be adopted.

Then, a targeting vector for homologous recombination for the used in the mutation of the smooth muscle myosin heavy chain gene may be created. The targeting vector for the homologous recombination may be designed by deleting a part of or an entire wild-type gene or substituting the gene with another non-homologous base sequence so as to have a base sequence homologous to the smooth muscle myosin heavy chain gene in an upstream or downstream region surrounding the part involving the deletion or the substitution for the other sequence (the non-homologous sequencing part).

When a reporter gene is to be inserted in a smooth muscle myosin heavy chain gene, a targeting vector can be designed so as to incorporate the reporter gene sequence in a non-homologous sequencing part. In this case, a targeting vector is preferably so designed as to make the translation frame of the reporter gene consistent with the translation frame of a smooth muscle myosin heavy chain gene.

Further, to this targeting vector, such a gene as neomycin-resistant gene (neo), herpes virus thymidine phosphorylated enzyme gene (HSV-tk), and diphtheria toxin A fragment gene (DT-A) which has been generally used for the selection of a drug is desirably inserted so as to allow the selection in terms of drug-resistance. These genes can be used as a marker gene for the selection of cells having a vector incorporated therein or cells having a high possibility of inducing a homologous recombination aimed at(Experimental Medicine, Bio Manual Series as a separate-volume supplement, Vol. 8, pages 43 - 82, 1995, written by Shinichi Aizawa and published by Yodosha K.K.). The targeting vector, in the case of containing a neo therein, for example, manifests resistance to an aminoglycoside type antibiotic like G418 and, in the case of containing a HSV-tk therein, manifests sensitivity to a nucleic acid derivative such as gancyclovir.

The targeting vector for the homologous recombination may be obtained by cloning a smooth muscle myosin heavy chain gene from a chromosome, cleaving the cloned gene with a proper restriction enzyme to obtain a fragment or amplifying this fragment as by a polymerase chain reaction (PCR) method to obtain an amplified DNA fragment, and binding (linking) the fragment or the amplified DNA fragment with a fragment containing a linker DNA synthesized by the use of a DNA synthesizer, a reporter gene, a drug-resistant marker gene, and the like in a appropriate order in accordance with the design as mentioned above. Any of the preparation procedure of a vector DNA for the homologous recombination can be carried out by the ordinary DNA recombination technique in vitro.

Subsequently, the targeting vector for the homologous recombination prepared as described above is introduced into proper cells. The cells to be used herein are preferably derived from an animal to be closely related to an animal intended to be produced and at the same time, used generally in the production of a chimera animal such as, for example, embryonic stem (ES) cells. As typical examples of the procedure of introducing DNA in a cell, ordinary methods including an electroporation method, a calcium phosphate method, a lipofection method, and a DEAE-dextran method may be cited. Among other methods cited above, an electroportation method is used preferably.

In some cells having a targeting vector for the homologous recombination incorporated therein, the smooth muscle myosin heavy chain gene of a chromosome from the incorporating cell induces the recombination with the targeting vector in two homologous sequence regions and consequently forms a variant gene locus which results from the substitution of the sequence surrounded by these regions with a sequence derived from the vector DNA.

Cells having the targeting vector incorporated therein can be selected by culturing the cells for a proper period in the presence of a drug based on the expression of a marker gene such as a drug-resistant of the vector DNA. From the selected cells, the cells which have induced the mutation in consequence of the homologous recombination may be analyzed and selected by a PCR method (Experimental Medicine, Bio Manual Series as a separate-volume supplement, Vol. 8, pages 83-103, 1995, written by Shinichi Aizawa and published by Yodosha K.K.) or a Southern blotting technique ("Embryologic Engineering Experiment Manual, "supervised by Tatsuji Nomura and edited by Motoya Katsuki, 1987, published by Kodansha K.K.) and thereafter confirmed the occurrence of the mutation aimed at. In this manner, embryonic stem cells of a non-human animal containing a variant gene as contemplated by this invention can be obtained.

According to another aspect of this invention, a chimera animal of this invention is provided by using cells in which a variant gene locus results from the method as described above.

The above aspect will be described specifically below.

The production of a chimera animal does not require to use any special method but may be implemented in accordance with any of methods in popular use such as an injection technique and an agglutination technique (see Cell Engineering, Experiment Protocol Series as a separate-volume supplement, Experiment Protocol on Molecular Medicine, pages 234-266, compiled by Tokyo University Medical Research Institute, 1995, published by Shujunsha K.K., for example).

To be specific, when a chimera mouse is expected to be produced by an agglutination technique, for example, an infant may be obtained by dissociating cells carrying a variant gene locus (for example, ES cells) by trypsinization, mixing the dissociated cells with an early embryo deprived of a clear zone (embryo at an 8-cell stage), culturing the resultant mixture, and transplanting the culture in an uterus of a pseudopregnant mouse. The infants obtained as described above may be screened by a PCR technique or a Southern blotting technique as mentioned above, or by the color of hair of infant and, when a reporter gene has been inserted, by the detection of the reporter gene product, to select an infant containing the variant gene aimed at. Thus, anon-human animal containing a variant gene according to this invention can be provided.

The chimera animal obtained in this manner, by being mated with an animal of a proper line of the same species, may produce an infant of a second generation. Then, a chimera mouse having germ cells derived from ES cells aimed at may be identified by using as an index a phenotype of this infant in the same manner as described above. By repeating this mating process, the pregnancy of the non-human animal containing the variant gene of this invention can be obtained.

In this case, from the infant of the chimera animal which has been obtained by mating the chimera animal obtained above with an animal of a proper line of the same species, an animal having a smooth muscle myosin heavy chain gene mutated heterologously can be obtained. By mating these two heterologously mutated animals,a homologously mutated animal can be obtained from the infant group. The fact that the animal has the smooth muscle heavy chain gene thereof heterologously or heterologously mutated can be confined by analyzing the chromosome DNA similarly by a PCR technique or a Southern blotting technique.

A method for breeding the animal produced by this invention is not specially limited but the animal can be bred by a method similar to that used for any normal animal.

According to still another aspect of this invention, cells which are obtained by isolating and culturing the non-human chimera animal obtained by the method described above or the pregnancy thereof is provided.

The term "cells" as used in this aspect means cells which are obtained by isolating and culturing a part of the body of the non-human chimera animal produced as described above or the pregnancy thereof. The cells may be either somatic cells or germ cells. Preferably, somatic cells, especially somatic cells like myeloma cells or smooth muscle cells which can be subjected to subcultivation is used.

The cells which have been isolated and cultured according to this invention can be cultured by using any of methods well-known in the art.

Now, this invention will be described more specifically below with reference to working examples.

### Experiment 1: Production of recombinant ES cells and mouse derived from ES cells

### (1) Cloning of a smooth muscle myosin heavy chain gene from a murine chromosome

This experiment used embryo stem cells from 129 type mice for the gene mutation. For the purpose of cloning a genomic gene of this type, a gene of a chromosomes containing a translation initiation point of a smooth muscle myosin heavy chain from another mice line was prepared and then digested with Pst I and Spe I to obtain about 0.8 Kb of a fragment.

A plaque hybridization technique was carried out in an usual manner by using as a probe about 0.8 Kb of the genome DNA fragment (labeled with ³²P by a random primer method) of the mouse smooth muscle myosin heavy chain, to clone a mouse smooth muscle myosin heavy chain gene aimed at from 129SV mouse genomic libraries (Stratagene). Three positive clones were obtained as a result of screening for about one million clones. When phage DNA's were prepared from these clones, all the clones were found to contain about 15 kb of Not I cleaved fragments. These fragments were each subcloned into a plasmid, Bluescript II SK- (Toyobo K.K.). When the base sequence of the inserted fragment in each the plasmid was examined, one of the three clones was found to have an inserted fragment having the identical sequence to that of the known mouse smooth muscle myosin heavy chain gene (Hasegawa et al., Biochemical and Biophysical Research Communications, Vol. 232, pages 313-316, 1997), to confirm that this clone certainly contained a fragment containing a translation initiation point of a smooth muscle myosin heavy chain gene aimed at.

### (2) Construction of a targeting vector for the homologous recombination

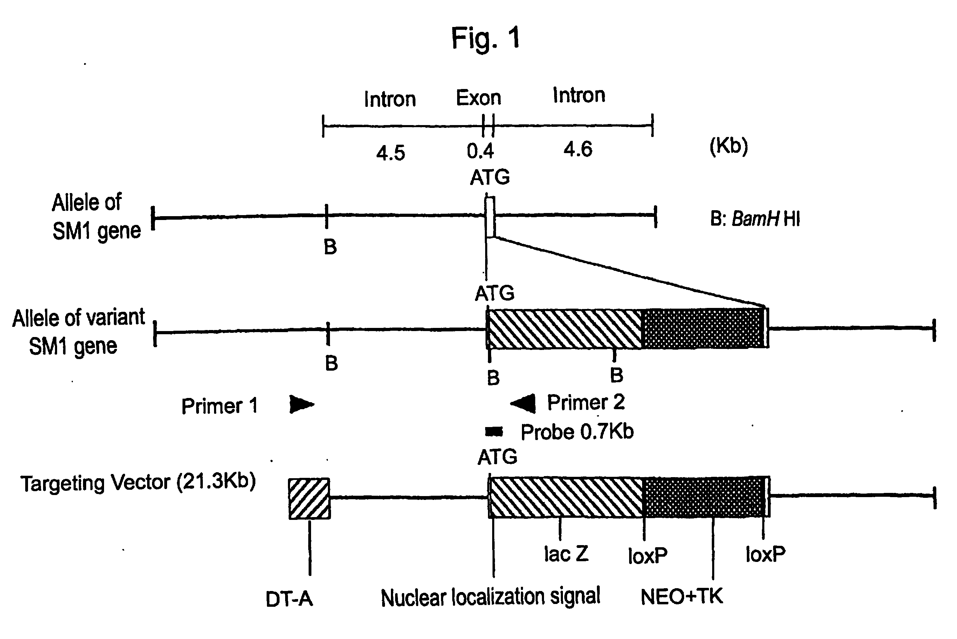

In this example, a targeting vector for the homologous recombination as shown in Fig. 1 was designed. In a mouse smooth muscle myosin heavy chain gene, an entire translation region coding for a smooth muscle myosin heavy chain from an initiation codon to a termination codon is not contained in one exon but is divided into a plurality of exons. The open square in an allele of the SM1 gene shown in Fig. 1 represents an exon containing a translation initiation point of a smooth muscle myosin heavy chain gene. The construction of a targeting vector was so designed as to prevent the expression of a smooth muscle myosin heavy chain protein while avoiding breakage of a transcription regulating system to the fullest possible extent.

The method for preparing a targeting vector will be described in detail below. The targeting vector for the homologous recombination which was produced in the present example has such a structure as that a translation frame of an E. coli's β-galactosidase gene ("lacZ" in Fig. 1) was connected to a downstream of an initiation codon of a smooth muscle myosin heavy chain gene in such a manner as to contact with a translation frame of SM1, a neomycin-resistant gene ("neo" in Fig. 1) and a herpes virus thymidine phosphorylated enzyme gene ("tk" in Fig. 1) were connected coordinately, about 4.5 kb of a homologous region of a smooth muscle myosin heavy chain gene was added to 5' side of the β-galactosidase gene and about 5 kb of a homologous region of a smooth muscle myosin heavy chain gene was also added across the herpes virus thymidine phosphorylated enzyme gene on the 3' thereof, and further a diphtheria toxin A fragment gene ("DT-A" in Fig. 1) was connected to 5' end of about 4.5 kb of a sequence, and a sequence of Bluescript plasmid (not shown) was connected to the upstream thereof as shown in Fig. 1. By the homologous recombination with this vector, a variant gene locus having lacZ, neo, and tk inserted therein was generated in the translation region of one of the smooth muscle myosin heavy chain gene alleles.

Now, the method for preparing a targeting vector for the homologous recombination according to the present example will be described below.

Firstly, for the purpose of inserting a reporter gene and a drug-resistant gene, the gene was mutated by using an U.S.E. Mutagenesis kit (Pharmacia) so as to obtain a cleavage site (Not I) immediately downstream of the translation initiation point of the smooth muscle myosin heavy chain gene which has been subcloned in the Bluescript plasmid.

Then, fragments destined to be inserted (namely, lacZ, neo, and tk) were sequentially linked and added in accordance with an ordinary method of gene manipulation using E. coli as a host by utilizing the Not I site thus obtained, and a diphtheria toxin A fragment gene was further linked to the outer of the homologous region on 5' side, to prepare about 21 kb of a targeting plasmid for the homologous recombination.

In this case, the targeting plasmid for the homologous recombination contained 34 mer of loxP sequences ("loxP" in Fig. 1) disposed on both sides of neo and tk in order to use a Cre-loxP System (Experimental Medicine, Bio Manual Series as a separate-volume supplement, Vol. 18, pages 71-82, 1995, written by Shinichi Aizawa and published by Yodosha K.K.). With this system, when a Cre protein derived from a bacteriophage P1 is expressed after the production of the homologous recombinant, the neo and tk can be removed.

The targeting plasmid for the homologous recombination thus obtained was cut with a restriction enzyme, Srf I, to linearize the plasmid, which was then used for the introduction into ES cells as described below.

### (3) Culture of mouse ES cells

ES cells were cultured on feeder cells in a culture medium supplemented with a leukemia inhibitory factor (LIF) for the purpose of maintaining the undifferentiated state thereof. The culture medium to be used herein for culturing the ES cells (hereinafter referred to simply as "ES culture medium") was produced by adding to Dulbecco's modified Eagle's medium (High glucose, Gibco; Code No. 11960-010) supplemented with 15% fetal bovine serum, 5 × 10⁴ U/ml of LIF (AMRAD), 10⁻⁴ M of 2-mercaptoethanol, non-essential amino acids (Gibco; Code No. 11360-013), sodium pyruvate (Gibco; Cote No. 11140-019), and L-glutamine (Gibco; Code NO. 25030-024) and further supplemented with potassium penicillin and streptomycin sulfate as antibiotics respectively in amounts to give final concentrations of 50 U/ml and 50 µg/ml. As the feeder cells, were used primary cultured embryonic fibroblast cells derived from a murine embryo (EF cells made by LifeTech Oriental K.K.) having the differentiation thereof stopped by the treatment with mitomycin C and cultured until they had reached confluence. The ES cells were plated on the feeder cells at a density of about 3 × 10⁶ cells per a 10 cm tissue culture grade culture petri dish, cultured in the above culture medium at 37°C in an atmosphere of 5% CO₂, and subcultured every two to three days. The culture medium was replaced daily with a fresh medium.

### (4) Preparation of variant ES cells by homologous recombination

About 1 × 10⁷ ES cells of the above (3) were suspended in about 0.8 ml of a buffer for the electroporation (Dulbecco's modified Eagle's medium). The resultant suspension, after adding thereto 10 µg of the linearized targeting vector DNA resulting from the above (2), was subjected to electroporation. This electroporation was performed by using a Gene Pulser (Bio Rad) under conditions of 210 V of field strength, 0.4 cm of path length, and 500 µF of capacitance.

After the electroporation was finished, the suspension was left standing at room temperature for 10 minutes. In a 10 cm tissue culture grade petri dish in which the feeder cells similar to those of the above (3) have been plated, this suspension was inoculated at a density of 2 to 4 × 10⁶ cells per a 10 cm tissue culture grade petri dish. After the electroporation was finished, the cells were cultured in an ES culture medium at 37°C in an atmosphere of 5% CO₂ for two days. Then, the culture medium was exchanged for an ES culture medium containing 300 µg/ml of G418. The cells were continuously cultured on neomycin-resistant EF cells (feeder cells) in the presence of a drug under the same conditions for 10 to 14 days with the culture medium replaced with a fresh medium daily. The drug-resistant colonies consequently formed were independently picked up under a microscope and divided into single cells by pipetting with a micropipette, which cells were independently cultured in a microplate. These cells were sequentially subcultured in a 24 well plate, then in a 6 well plate, and subsequently in a 10 cm petri dish, to proliferate the cells. A chromosome DNA was prepared from a part of these cells.

The chromosome DNA prepared from the ES cells from the drug-resistant colony was assayed by a PCR technique to determine whether or not the homologous recombination was generated. The assay by a PCR technique was carried out substantially in accordance with the known method described, for example, in Experimental Medicine, Bio Manual Series as a separate-volume supplement, vol. 8, pages 83-103, 1995, written by Shinichi Aizawa and published by Yodosha K.K, as follows. To be specific, as the primers used for the PCR, two primers illustrated in Fig. 1, i.e., a primer 1 corresponding to the upstream sequence from the homologous region on the 5' side of the targeting vector for the homologous recombination and a primer 2 corresponding to the lacZ sequence in the targeting vector, were synthesized with a DNA synthesizer. The base sequences of the primer 1 and the primer 2 mentioned above were as follows.
Primer 1: 5'-TGCAGGTTCATAGGAGCAGAG-3'
Primer 2: 5'-GCGGATTGACCGTAATGGGATAGG-3'

By using the chromosome DNA of each of the ES cells, total 35 cycles of the PCR was carried out with LA-PCR kit Ver. 2 (Takara Shuzo Co., Ltd.), one cycle of which comprises denaturation of DNA (98°C, 20 seconds), and annealing of primer and elongation of primer (65°C, 12 minutes). Finally, one cycle of the elongation procedure (72°C, 10 minutes) was further performed for the purpose of elongating the primer. Thereafter, the resultant sample was subjected to agarose gel electrophoresis.

As a result of the assay by the PCR method mentioned above, the agarose gel electrophoresis carried out subsequently to the PCR detected about 5 kb of bands in the chromosome DNAs of six ES cells of 240 drug-resistant colonies. It was consequently inferred that the mutation of a smooth muscle myosin gene by the homologous recombination occurred in these ES cells.

The six ES cells were further analyzed by a Southern blotting technique to confirm that the homologous recombination had occurred therein. The analysis by the Southern blotting technique was carried out as follows substantially in accordance with the method described, for example, in the "Embryologic Experiment Manual," supervised by Tatsuji Nomura, compiled by Motoya Katsuki, 1987, published by Kodansha K.K.

The smooth muscle myosin heavy chain genes of the ES cells both of the wild type and the variant type were examined by a Southern blotting technique. This examination was carried out as follows, with about 0.7 kb of a fragment illustrated in Fig. 1 as a specific probe. A genome DNA was prepared from cultured ES cells, digested with a restriction enzyme, Bam HI, and then subjected to electrophoresis with 0.8% agarose gel, and transferred to a nylon membrane (Amersham; trademark designation of "Hybond-N+"). The nylon membrane after being dried was kept shaken in 50 ml of a hybridization solution (5 × SSPE / 5 × Denhart's solution / 0.5% SDS / 20 µg/ml of salmon sperm DNA) at 65°C and a DNA probe labeled with ³²P added thereto were left reaction overnight. After the reaction was completed, the nylon membrane was washed in 2 × SSPE containing 0.1% SDS at 65°C for 10 minutes, in 1 × SSPE containing 0.1% SDS at 65°C for 15 minutes, and in 0.1 × SSPE containing 0.1% SDS at 65°C for 15 minutes. The treatment was followed by autoradiography.

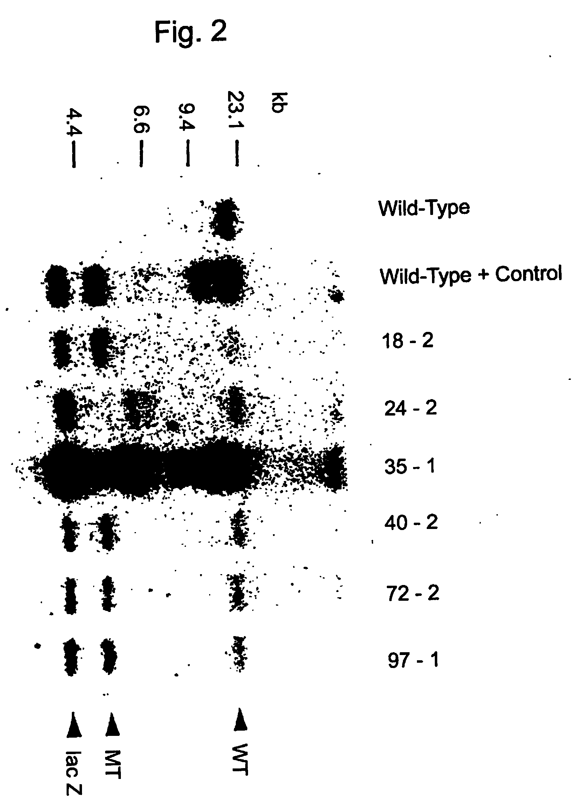

The results by Southern blotting of the product digested with a restriction enzyme, Bam HI, are shown in Fig. 2. It is clearly noted from Fig. 2 that about 20 kb of the wild type DNA was solely detected in the wild type ES cells and that in four (18-2, 40-2, 72-2, and 97-1) of the six ES cells, about 4.0 kb and about 4.5 kb of the variant fragments were detected as well as about 20 kb of the wild type fragment. From these results, it was confirmed that the four variant ES cells designated as 18-2, 40-2, 72-2, and 97-1 were variant ES cells that had undergone the homologous recombination aimed at.

### (5) Production of chimera mouse from variant ES cells

The production of a chimera mouse using ES cells containing a variant smooth muscle myosin heavy chain gene was attempted on each the four variant ES cells obtained in (4) above by using an agglutination technique (Cell Engineering, Experiment Protocol Series as a separate-volume supplement, Experiment Protocol on Molecular Medicine, pages 234-266, compiled by Tokyo University Medical Research Institute, 1995, published by Shujunsha K.K. and Gene Targeting:A practical Approach, written by A.L. Joyner, 1993, IRL PRESS) as follows.

The chimera mouse was produced by mixing an early embryo deprived of a clear zone (embryo at an 8-cell stage) with ES cells which had been dissociated by trypsinization and allowing them to develop together. The embryo at an 8-cell stage was obtained by superovulating a mouse for the collection of ova, extracting and collecting the oviduct and the uterus therefrom. The embryos at an 8-cell stage thus collected was transferred into an acidic Tyrode's solution to resolve the clear zone. The ES cells used for the agglutination was treated with trypsin so as to form a cell cluster of a certain degree (10 to 15 cells). Then, the cells were plated on a gelatin-coated petri dish and left standing in a CO₂ incubator (37°C, 5% CO₂) for 10 to 15 minutes. Then, the cells which had not adhered to the petri dish were collected. A chimera embryo was prepared from two embryos deprived in advance of a clear zone by agglutinating these two embryos with an ES cluster in such a manner as to put the ES cluster between the embryos, incubating them together in a CO₂ incubator (37°C, 5% CO₂) overnight.

Then, the embryo cultured as described above was transplanted to an uterus as follows. Firstly, a pseudopregnant mouse was given a cut in the back to extract the uterus therethrough. Then, the chimera embryo which had been incubated overnight as described above was transplanted in the extracted uterus. The uterus was returned into the body of the mouse, with the cut sutured. Thereafter, the mouse was generally delivered spontaneously, except that when it failed to bear an infant on the expected day (normally 17 days after the transplantation), the infant was extracted by Caesarean operation. The infant thus born by the mouse was bred by a foster parent as usually practiced.

As a result, 47 infants were obtained from 998 transplanted embryos. When these infants were examined as to the color of hair (in the case of a chimera mouse, hair assuming a color other than black was come out), 11 of the 47 infants were judged by its hair color to be chimera mice.

### (6) Production of progeny from chimera mouse

The chimera mouse obtained in (5) above were mated with a C57BL/6J type mouse to examine whether or not they would bear an infant derived from the ES cells. When the germ cells of a given chimera mouse has been derived from the ES cells, the infant to be born grows hair of a color other than black. When not less than 1500 infants were obtained from 11 lines of chimera mice, seven of them grew hair of brown color. The genes of these infants were confirmed by a PCR method, to find that the genes of four of them were of a variant type. It is confirmed by the results that some ES cells were differentiated to a germ line of the chimera mouse thus produced.

Heterozygous mice containing a variant smooth muscle myosin heavy chain gene were produced from the chimera mice as described above.

Further, from the infants of brown-colored hair which were obtained by mating the chimera mouse confirmed to have the ES cells differentiated to the germ line as mentioned above with the C57BL/6J type mouse, heterozygous mice containing a variant type smooth muscle myosin heavy chain gene were obtained. The discrimination between the wild-type mice and the heterozygous mice was effected by extracting a chromosome DNA from the cells of the tail of a given mouse and subjecting this chromosome DNA to PCR using a separate primer or Southern hybridization in accordance with the procedure mentioned above. The mouse which was found by either of these techniques to carry a detectable variant fragment and further manifested β-galactosidase activity was judged to be a heterozygous mouse (for further details, refer to Example 2 below).

### Experiment 2: Identification of cells expressing reporter gene of heterozygous mouse

The tail was cut off from the heterozygous mouse obtained in Experiment 1, and incubated and fixed in a fixing solution (2% formaldehyde and 0.2% glutaraldehyde) at room temperature for 10 minutes. The fixed tail was washed with PBS and then incubated with a X-Gal reaction solution (5 mM potassium ferricyanide, 5 mM potassium ferrocyanide, 2 mM magnesium chloride, 1 mg/ml X-Gal, and PBS) at 37°C for 0.5 hour to overnight. As a result, the tail was confined to be dyed in a blue color specifically in the smooth muscle cells thereof which shows the reporter (β-galactosidase) activity.

### Experiment 3: Detection of reporter activity during induction of differentiation of recombinant

### ES cells in vitro

The induction of differentiation of recombinant ES cells was carried out in accordance with the technique proposed by Drab, M. et al. (FASEB J., 11, 905-915, 1997). The recombinant ES cells 40-2 obtained in Experiment 1 (4) were cultured on supporting cells until they had reached confluence and then treated with 0.25% trypsin and 0.04% EDTA to have the recombinant ES cells detached therefrom. Then, the resultant cells were plated at a density of about 4 × 10⁶ on each of gelatin-coated petri dishes. The cells were cultured in an ES culture medium at 37°C in an atmosphere of 5% CO₂ for two days until they had reached confluence, washed with PBS, and treated with 0.25% trypsin and 0.04% EDTA. They were left standing at room temperature for one to two minutes. When the cells were detached from the petri dish, they were combined with 10 ml of a culture medium (DMEM and 10% fetal bovine serum). The resultant cell suspension was transferred into a plate for culture of microorganism. At this time, the cell suspension in one 10 cm petri dish was dispensed to three culturing plates. The cells failed to adhere to the plates and formed a cell cluster. The culture was continued for seven days, with the culture medium replaced with a fresh medium every two days. The cell clusters were collected by spontaneous sedimentation. The collected clusters and a fresh medium added thereto were together seeded in a 24 well gelatin-coated culture plate. The clusters were plated in a well so as to give 2 - 3 cell clusters in each well. On the following day, the cell clusters to which 10⁻⁸ M retinoic acid and 0.5 × 10^{-³} M dibutyryl-cyclic AMP had been added were cultured for five days. The cell clusters were transferred to an ordinary culture medium and cultured thereon at 37°C in an atmosphere of 5% CO₂ for 12 days. Meanwhile, the culture medium was replaced with a fresh medium once per four days. The cells thus induced by differentiation were dyed with X-gal by a well-known method (Experimental Medicine, Bio Manual Series as a separate-volume supplement, Gene Targeting, written by Shinichi Aizawa and published by Yodosha K.K., 1995).

As a result, it was confirmed that some differentiated ES cells from the ES cells manifested β-galactosidase activity.

### Experiment 4: Isolation and culture of myeloma cells

A thigh bone of the chimera mouse obtained in Experiment 1 (5) was excised out and the opposite ends of the thigh bone were cut off with scissors. Clusters of myeloma cells were acquired by injecting 5 ml of a culture medium (an αMEM culture medium containing 10% fetal bovine serum and 50 mM 2-mercaptoethanol) to the cut end of the bone by the use of a 1 ml injection syringe fitted with a 25G injection needle and the content in the syringe was blown out. The cluster of cells was dissociated through a 1 ml grade injection syringe. The dissociated cells were transferred to a culturing plate for tissue culture and cultured therein with the same culture medium in a CO₂ incubator (5% CO₂) at 33°C. The floating cells were removed by weekly replacement of the culture medium with a fresh medium. The interstitial cells which adhered to the plate were cultured as myeloma cells.

### Experiment 6: Production of SM1 reporter myeloma cell strain

The SV40 large T antigen gene used in the present experiment was prepared as follows. As a plasmid for expressing a temperature-sensitive SV40 large T antigen gene, pSVtsA58ori(-) reported by Tatewaki et al. (Jpn. J. Cancer Res., 82: 1344, 1991) was used. This plasmid was cleaved with a restriction enzyme, Bgl I, and about 7 kb of a fragment containing a SV40 large T antigen gene consequently obtained was used as a fragment for the introduction of gene.

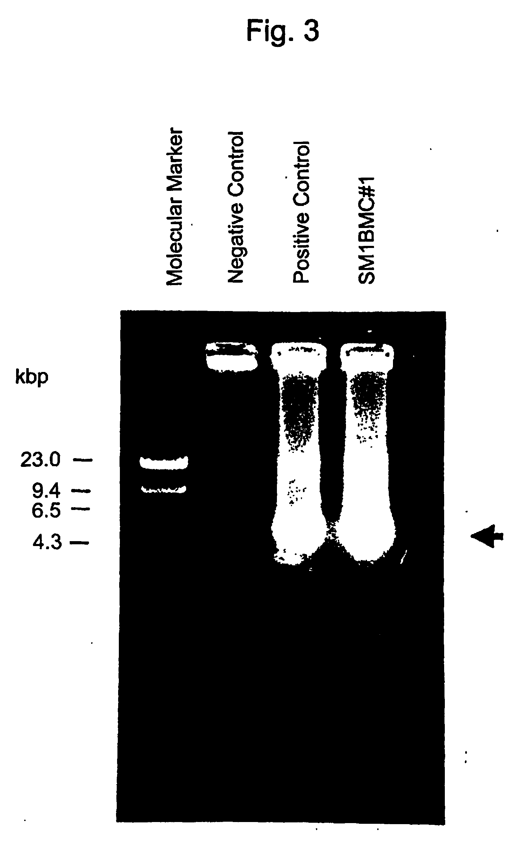

Cell strains were obtained by introducing the temperature-sensitive SV40 large T antigen gene prepared as described above into the interstitial cells of Experiment 4 which had been cultured for about one month. The introduction of the gene into the cells was carried out by the use of Lipofectamine (Gibco BRL Corp.) in accordance with the protocol annexed thereto. Generally, about 9 mg of gene was used per culture plate (about 1 × 10⁵ cells) of 6 cm in diameter. The cells which formed colonies within three weeks after introducing the gene were isolated by the use of a pipet and designated as "Cloned Cell Strain SM1BMC#1". The presence of the SM1 reporter gene which had undergone homologous recombination was confirmed by a PCR reaction. Generally, a standard PCR reaction was performed with the LA Tag DNA polymerase (TAKARA K.K.) by using the chromosome gene of SM1BMC#1 purified with a QIAGEN Blood & Cell culture DNA kit (QIAGEN Corp.) and using as a primer an oligomer DNA of a DNA sequence (described in the above-mentioned example) which had been designed so as to amplify solely the sequence of the SM1 reporter gene. The results of this experiment are shown in Fig. 3. The PCR reaction solution was subjected to agarose gel electrophoresis and dyed with ethidium bromide. The SM1BMC#1 was confirmed to have amplified 5.1 kb of a band showing the presence of DNA of the SM1 reporter gene. On other words, the chromosome of the SM1BMC#1 was confirmed by homologous recombination to contain a SM1 reporter gene.

The variant myeloma cells, SM1BMC#1, thus obtained were nationally deposited in National Institute of Bioscience and Human-Technology Agency of Industrial Science and Technology on March 6, 1998 under the Accession No. P-16687 and this deposition was changed to the international deposition in accordance with the Budapest Treaty in National Institute of Bioscience and Human-Technology Agency of Industrial Science and Technology on January 20, 1999 under the Accession No. BP-6625.

### Experiment 6: Determination of β-galactosidase activity

The SM1BMC #1 of Experiment 5 which had been cultured in a culture plate for tissue culture was washed with PBS and then fixed by being incubated in a fixing solution (2% formaldehyde and 0.2% glutaraldehyde) at room temperature for 10 minutes. It was washed with PBS and then incubated in a X-Gal reaction solution (5 mM potassium ferricyanide, 5 mM potassium ferrocyanide, 2 mM magnesium chloride, 1 mg/ml X-Gal, and PBS) at 37°C for 4 hours to overnight. The determination of β-galactosidase activity was carried out by washing the cells with PBS, changing the PBS to a preservation solution (4% formaldehyde), counting cells to be dyed in blue as positive cells under a microscope. As a result, the presence of positive cells was confirmed.

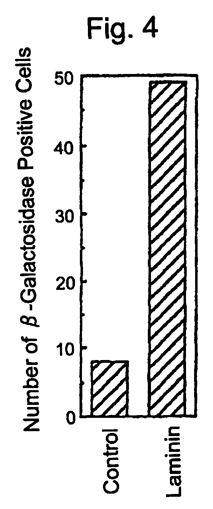

It has been reported that when smooth muscle cells which have been mechanically stimulated is cultured on a culture plate coated with laminin or collagen, the expression of SM1 is varied (Cir. Res. 79 (5), 1046-1053, 1996). In the light of this phenomenon, it was examined about whether or not the expression of SM1 was varied when the SM1BMC#1 was cultured on a laminin-coated culture plate. The results of this trial are shown in Fig. 4. It is clearly noted from Fig. 4 that for the SM1BMC#1 which was cultured on the laminin-coated culture plate, more positive cells were observed as compared with that cultured on a control culture plate, indicating that the change of the expression of SM1 due to the stimulation can be assayed in terms of β-galactosidase activity.

### Industrial Applicability

As described above, the cell or the animal of this invention is characterized by containing a variant smooth muscle myosin heavy chain gene incapable of substantially functioning as a smooth muscle myosin heavy chain gene derived from a chromosome. The cells or the animal of this invention, therefore, can be expected to provide a new study material or pathologic model to be used in biomedical study concerning a role of a smooth muscle myosin heavy chain gene as in the blood vessel, other unknown physiological functions of a gene, and a mechanism for the expression of a gene, and the like. The animal which contains a variant gene having a smooth muscle myosin heavy chain gene mutated and, at the same time, having such a reporter gene as β-galactosidase gene inserted therein, for example, has such outstanding characteristics as follows. Specifically, for the heterozygous variant animal, expressed cells can be detected by using as an index the expression of a reporter gene in a variant gene located on one of chromosome gene loci (for example, by the coloration of a dye as a substrate when the reporter gene is β-galactosidase gene) and, at the same time, it can be studies about the regulation of the expression of a functional smooth muscle myosin heavy chain gene which is maintained on the another chromosome gene locus.

Further, the cells or the animal according to this invention can be expected to provide a convenient test system for the evaluation of a new pharmaceutical preparation against arteriosclerosis, the research for biological availability or effects of such a compound as conventional compounds against arteriosclerosis.

## Claims

1. Embryonic stem cells of a non-human animal or the cells derived therefrom which contain a variant smooth muscle myosin heavy chain gene incapable of substantially functioning as a smooth muscle myosin heavy chain gene derived from a chromosome.

2. Embryonic stem cells or the cells derived therefrom according to claim 1, wherein said variant gene is obtained by deleting at least a part of the base sequence for the smooth muscle myosin heavy chain gene derived from the chromosome, by inserting a foreign gene into the smooth muscle myosin heavy chain gene derived from the chromosome, by substituting at least a part of the smooth muscle myosin heavy chain gene derived from the chromosome with a foreign gene, or by the combinations thereof, whereby the function of the smooth muscle myosin heavy chain gene derived from the chromosome is substantially lost.

3. Embryonic stem cells or the cells derived therefrom according to claim 2, wherein said variant gene is obtained by inserting a reporter gene in the smooth muscle myosin heavy chain gene derived from the chromosome.

4. Embryonic stem cells or the cells derived therefrom according to claim 3, wherein said reporter gene is a β-galactosidase gene.

5. Embryonic stem cells or the cells derived therefrom according to any one of claims 1 to 4, wherein said non-human animal is a rodent.

6. Embryonic stem cells or the cells derived therefrom according to claim 5, wherein said rodent is a mouse.

7. A non-human animal or the progeny thereof which contains a variant smooth muscle myosin heavy chain gene incapable of substantially functioning as a smooth muscle myosin heavy chain gene derived from a chromosome.

8. A non-human animal or the progeny thereof according to claim 7, wherein said variant gene is obtained by deleting at least a part of the base sequence for the smooth muscle myosin heavy chain gene derived from the chromosome, by inserting a foreign gene into the smooth muscle myosin heavy chain gene derived from the chromosome, by substituting at least a part of the smooth muscle myosin heavy chain gene derived from the chromosome with a foreign gene, or by the combinations thereof, whereby the function of the smooth muscle myosin heavy chain gene derived from the chromosome is substantially lost.

9. A non-human animal or the progeny thereof according to claim 8, wherein said variant gene is obtained by inserting a reporter gene in the smooth muscle myosin heavy chain gene derived from the chromosome.

10. A non-human animal or the progeny thereof according to claim 9, wherein said reporter gene is a β-galactosidase gene.

11. A non-human animal or the progeny thereof according to any one of claims 7 to 10, wherein said non-human animal is a rodent.

12. A non-human animal or the progeny thereof according to claim 11, wherein said rodent is a mouse.

13. Cells which are obtained by separating and culturing from the non-human animal or the progeny thereof set forth in any one of claims 9 to 12.

14. A cell line which is obtained by introducing a T antigen gene into the cells set forth in claim 13.

15. A cell strain according to claim 14, which is a myeloma cell line deposited as "SM1BMC #1" under the accession No. FERM BP-6625.
